**Europäisches Patentamt**

**European Patent Office**

**Office européen des brevets**

(11) Numéro de publication: **0 354 828**
**A1**

(12) **DEMANDE DE BREVET EUROPEEN**

(21) Numéro de dépôt: **89402136.9**

(22) Date de dépôt: **27.07.89**

(51) Int. Cl.⁵: **C 12 P 7/56**

(30) Priorité: **10.08.88 FR 8810789**

(43) Date de publication de la demande:
**14.02.90 Bulletin 90/07**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevole Cédex (FR)**

(72) Inventeur: **Schneider, Didier**
**7, rue Eloi Ricard**
**F-79500 Melle (FR)**

**Lamonerie, Hubert**
**rue Foucaudrie**
**F-79500 Melle (FR)**

(74) Mandataire: **Fabre, Madeleine-France et al**
**RHONE-POULENC CHIMIE Service Brevets Chimie 25,**
**quai Paul Doumer**
**F-92408 Courbevoie Cédex (FR)**

(54) Procédé de production d'acide lactique.

(57) Procédé de production d'acide lactique par fermentation d'hydrates de carbone au moyen de microorganismes. Le procédé est caractérisé en ce que la fermentation est réalisée dans un milieu nutritif contenant de l'amidon comme source de carbone assimilable en présence d'une enzyme de saccharification.

**Description**

## PROCEDE DE PRODUCTION D'ACIDE LACTIQUE

La présente invention a pour objet un procédé amélioré pour la production d'acide lactique par fermentation d'hydrates de carbone au moyen de microorganismes. Elle concerne plus particulièrement un procédé microbiologique par conversion de sucres dérivés de l'amidon.

La synthèse d'acides organiques par fermentation de sucres en présence d'un microorganisme approprié est universellement connue. Des acides typiques de telles fermentations microbiennes comprennent notamment l'acide acétique, l'acide lactique, l'acide citrique, les acides gluconique et 2-cétogluconique, l'acide fumarique, l'acide itaconique. Ces acides sont utiles dans les industries agroalimentaires, pharmaceutiques, chimiques et autres. Des procédés de préparation sont décrits par exemple dans l'ouvrage "Chemicals by fermentation" S.J. Gutcho-Noyes Data Corporation 1973.

Dans les fermentations industrielles, le choix du substrat hydrocarboné est basé à la fois sur sa disponibilité, sur son coût et sur son aptitude à permettre des productivités élevées. L'amidon a souvent été préconisé comme source de carbone bon marché. Cependant, tous les microorganismes ne sont pas capables de métaboliser l'amidon alors que la majorité d'entre eux métabolisent le dextrose. En conséquence, l'amidon doit être hydrolysé/saccharifié avant la fermentation. Il en résulte un coût de production accru.

La présente invention a pour but principal de proposer un procédé de fermentation économique utilisant l'amidon comme source de carbone nutritive et ayant une productivité au moins égale à celle obtenue avec le glucose ou les hydrolysats d'amidon riches en glucose.

Il a été trouvé que l'acide lactique pouvait être synthétisé en effectuant la fermentation au moyen du microorganisme producteur simultanément à l'hydrolyse enzymatique de l'amidon. Outre le gain de temps résultant de la suppression de l'étape préalable de saccharification de l'amidon, la vitesse de production de l'acide, de manière inattendue, n'est pas affectée comparativement à l'emploi d'un substrat de glucose, même si les conditions de la fermentation (pH et température) sont éloignées des conditions optimales d'activité de l'enzyme hydrolysante.

Selon l'invention, le procédé de production d'acide lactique par fermentation à l'aide de mircoorganismes d'un milieu nutritif aqueux contenant de l'amidon comme source de carbone assimilable est caractérisé en ce que la fermentation est conduite en présence additionnelle d'au moins une enzyme amylolytique saccharifiante.

L'amidon utilisé comme source de carbone selon l'invention peut être n'importe quel amidon de céréales comme l'amidon de blé, de maïs, de sorgho, de riz, de tapioca, de seigle, d'avoine ou un amidon de tubercules comme l'amidon de pomme de terre. L'amidon peut être mis en oeuvre sous forme brute, liquéfié ou partiellement saccharifié.

Le terme "amidon" inclut selon la présente description l'amidon brut en dispersion aqueuse et les hydrolysats d'amidon résultant de l'hydrolyse incomplète de l'amidon comme l'amidon fluidifié (liquéfié), les sirops d'amidon et les hydrolysats riches en dextrose. Les hydrolysats d'amidon diffèrent entre eux par leur degré d'hydrolyse, exprimé en équivalent dextrose D.E. et leur contenu en oligosaccharides et polysaccharides plus élevés. Les amidons fluidifiés présentent un D.E. compris entre environ 3 et 20 et renferment généralement 50 à 95 % de polysaccharides, de degré de fermentation supérieur à G 7 (7 unités glucose). Les sirops d'amidon ou sirops de glucose de faible équivalent dextrose présentent un D.E. allant de environ 20 à 68 avec 10 à 50 % de polysaccharides supérieurs à G 7. Les hydrolysats d'amidon ou sirops riches en dextrose ont un D.E. pouvant atteindre 90-98 %. La préparation des hydrolysats d'amidon est bien connu dans la technique. Conventionnellement, les amidons fluidifiés et les sirops d'amidon sont obtenus par hydrolyse acide et/ou enzymatique au moyen d'une α amylase de liquéfaction, éventuellement suivie d'une β amylase. Pour les hydrolysats riches en glucose, l'amidon est le plus souvent transformé selon un procédé en 2 étapes, par action d'une α amylase de liquéfaction puis par action d'une enzyme de saccharification comme la glucoamylase, connue aussi sous le nom d'amyloglucosidase.

Dans la pratique du procédé selon l'invention on utilisera de préférence un sirop d'amidon et encore plus préférentiellement un amidon fluidifié. L'emploi des hydrolysats riches en glucose n'est pas intéressant du point de vue économique étant donné qu'il implique une saccharification préalable qui est une étape longue, même dans les conditions d'activité maximales de l'amyloglucosidase qui se situent à une température supérieure à 50°C et à pH de 4,5-5.

L'amidon et ses produits d'hydrolyse peuvent être employés directement dans le procédé de l'invention sous forme non purifiée, après stérilisation. On peut bien entendu mettre en oeuvre des produits commerciaux purifiés, concentrés ou déshydratés comme les maltodextrines.

L'amidon est présent dans le milieu de fermentation en quantité nécessaire pour fournir environ 0,9 à 18 % en poids de glucose par rapport au milieu de fermentation. Exprimé en amidon brut, sur une base sèche, des quantités convenables peuvent être comprises entre 10 et 200 g/l, de préférence entre 60 et 160 g/l du milieu de fermentation.

Les enzymes amylolytiques de saccharification que l'on ajoute selon le procédé de l'invention dans le milieu de fermentation contenant le microorganisme producteur d'acide itaconique sont capables de transformer les dextrines de l'amidon en glucose et maltose. Comme enzymes de saccharification on peut citer les α amylases saccharifiantes comme l'α amylase de <u>Bacillus subtilis var.amylosaccharitiens</u>, l'α amylase fongique, les β amylases, la glucoamylase, l'isoamylase, la pullulanase. Ces enzymes peuvent être employées seules ou en mélange entre elles.

On donne la préférence à la glucoamylase du fait de sa haute spécificité. La glucoamylase peut être toute glucoamylase fongique telle que celles appartenant aux genres Aspergillus, Endomyces ou Rhizopus. Dans le cas notamment où l'on utilise l'amidon brut comme source de carbone, il est encore possible d'employer un enzyme liquéfiante en addition à l'enzyme saccharifiante, par exemple un mélange $\alpha$ amylase liquéfiante - $\beta$ amylase ou $\alpha$ amylase liquéfiante -glucoamylase. Des préparations enzymatiques industrielles sont décrites dans l'ouvrage Encycl. of Pol. Sc. Vol 6, pp 46-53.

L'enzyme amylolytique de saccharification, éventuellement de liquéfaction, est ajoutée dans le milieu de fermentation en quantité nécessaire pour effectuer la saccharification, respectivement la fluidification, de l'amidon. La quantité minimale utile est fonction de l'activité de l'enzyme, de la quantité et du D.E. de l'amidon présent dans le milieu et peut être déterminée facilement par l'homme de métier. De manière générale, on utilise des quantités suffisantes pour fournir 0,04 à 2 unités d'activité enzymatique, de préférence 0,1 à 1 unité, par gramme d'amidon (exprimé en matières sèches). A titre d'exemple d'enzyme saccharifiante, l'AMG 200 L®, commercialisée par NOVO INDUSTRY et qui est une glucoamylase, peut être ajoutée en quantité de 0,02 à 1 %, de préférence entre 0,05 et 0,5 % en poids basé sur le poids de solides contenu dans l'amidon liquéfié présent dans le milieu de fermentation.

N'importe quel microorganisme ayant la capacité de produire l'acide lactique D- ou L+ en présence de sucres peut être utilisé dans le procédé de l'invention. On peut notamment citer les bactéries appartenant au genre Lactobacillus telles que L. delbruckii, L. lactis, L. leichmanii, L. bulgaricus, L. jugurt, L. casei, L. italicus, L. plantarum ; au genre Steptococcus telles que S. thermophylus, S. faecium ; au genre Pediococcus telle que P. pentosaceus, aux germes Bacillus et Sporolactobacillus et des champignons tels que Rhizopus orizae.

En dehors de la source de carbone et de l'enzyme amylolytique utilisées selon l'invention, le milieu de production et les conditions de la fermentation peuvent être choisis parmi ceux décrits dans la littérature. Des milieux de production convenables sont décrits par exemple dans l'ouvrage Chemicals by fermentation déjà cité et dans de nombreux brevets, par exemple, US-A-3 125 494, FR-A-1 356 647, EP-A-69 291, EP-A-72 010, EP-A-113 215, US-A-4 564 594.

La source d'azote peut notamment être choisie parmi les composés organiques et/ou minéraux métabolisables, tels que l'extrait soluble de maïs (CSL) et/ou de soja, l'urée, la levure de bière, les peptones, les protéolysats de poisson, le sulfate d'ammonium, chlorure d'ammonium, phosphate d'ammonium, nitrate d'ammonium, etc... et leurs mélanges. Le milieu contient en outre des sels minéraux tels que sulfates, chlorures, phosphates de Ca, Mg, Na, K, Fe, Ni, Co, Cu, Mn, Zn, ainsi que des vitamines d'autres additifs conventionnels tels que des agents de contrôle de pH et/ou des agents anti-moussants.

Le microorganisme est introduit dans le milieu de fermentation de manière connue en soi à l'aide d'inoculums ou de cultures d'inter médiaires.

L'enzyme amylolytique est, de préférence, ajoutée dans le milieu stérile immédiatement avant inoculation. La fermentation est convenablement réalisée à un pH pouvant aller de environ 3,0 à 8,0, de préférence de 5,0 à 7,0 et à une température d'environ 20°C à environ 50°C, les conditions optimales dépendent de la souche particulière du microorganisme employé. Des pourcentages élevés d'acide lactique ont été obtenus en utilisant Lactobacillus lactis à un pH préférentiellement compris entre 5,5 et 6,0, la température étant maintenue à environ 35 à 45°C. L'agent neutralisant qui permet d'ajuster le milieu au pH convenable est choisi parmi les hydroxydes ou carbonates alcalins, alcalino-terreux ou d'ammonium et peut être introduit avant inoculation ou à tout stade de la fermentation, en continu ou en discontinu.

Après achèvement de la fermentation, l'acide lactique produit peut être récupéré du moût et purifié selon les méthodes connues telles que filtration, concentration, cristallisation ou extraction par solvants.

On comprendra que la description ci-dessus vise un procédé spécifique pour préparer de l'acide lactique par fermentation à l'aide de microorganismes d'un milieu contenant une source d'hydrate de carbone produite à partir d'amidon, en présence d'enzymes capables d'hydrolyser l'amidon ou ses produits de dégradation en mono- et disaccharides et que l'invention elle-même ne se limite pas à la composition précise du milieu de fermentation ni aux modes de réalisation particuliers.

Les exemples suivants illustrent l'invention.

<u>Exemple 1</u>

<u>a) Préparation d'amidon brut fluidifié</u>

Dans un fermenteur de capacité utile 50 litres, on introduit 6,75 kg d'amidon de blé (Ets ROQUETTE). La mise en suspension est effectuée à l'eau potable sous agitation dans un volume total de 20 litres. Le pH de la suspension est ajusté à 6,5 par $H_2SO_4$ et l'on ajoute 3,65 ml d'une préparation enzymatique contenant une $\alpha$ amylase thermostable (TERMAMYL 120L® - NOVO Industry) ). La suspension d'amidon est fluidifiée par injection de vapeur pendant 30 mn à 100°C, puis refroidie à température ambiante.

<u>b) Fermentation</u>

Dans le fermenteur contenant l'amidon fluidifié, on introduit 1,750 kg d'eau de lavage de maïs concentrée (CSL), 0,270 kg de protéolysat de poisson, 25 ml d'acide phosphorique. Le mélange est dilué à l'eau potable à un volume de 27 litres. La solution est agitée, ajustée à pH 6,0 par NaOH et stérilisée par injection de vapeur pendant 1 h à 100°C.

Dans un stérilisateur annexe, on dilue 4,2 kg de carbonate de calcium dans 13 l d'eau potable. La solution est stérilisée sous agitation par injection de vapeur pendant 1h30, refroidie et transférée stérilement dans le fermenteur. La température est ajustée à 40°C. On ajoute 15,6 ml d'une préparation enzymatique contenant une glucoamylase (AMG 200L® - NOVO Industry). Le milieu est ensemencé par 1,7 l d'une culture de Lactobacillus lactis ATCC 12314 préalablement incubée dans un milieu MRS (Milieu de De Man, Rogosa et Sharpe - réf. 0881-01 de DIFCO). Le milieu de fermentation, ayant un volume final de 50 litres, est agité et incubé sous atmosphère d'azote stérile à 40°C.

La durée de la fermentation est de 45 heures.

On obtient 123,3 g d'acide lactique D, soit une productivité de 2,74 g/l/h.

Exemples 2 à 4 (comparatif)

On réalise une série de fermentations de la manière décrite à l'exemple 1 b) à l'aide de la même culture de Lactobacillus lactis ATTC 12314 mais en l'absence de glucoamylase. La source de carbone est constituée de
- 6,50 kg de glucose monohydraté (exemple 2)
ou - 9,150 kg d'hydrolysat d'amidon (74968 commercialisé par les Ets ROQUETTE) ayant un contenu en matières sèches de 71 % en poids et un DE d'approximativement 96-98 (exemple 3)
ou - 6,75 kg d'amidon brut fluidifié dans les conditions décrites à l'exemple 1 (exemple 4)

Dans chaque exemple, le volume final du milieu de fermentation est de 50 litres.

La durée des fermentations et la productivité sont répertoriées dans le Tableau 1.

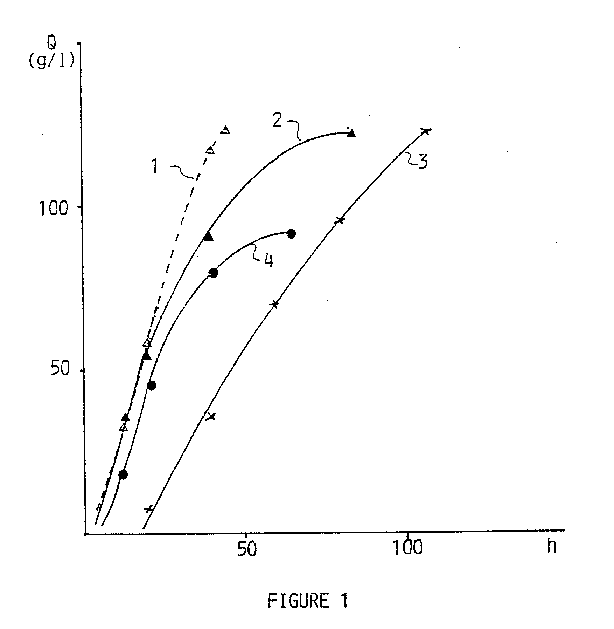

Au cours de chacun des exemples 1 à 4, des échantillons de moût sont prélevés périodiquement pour détermination de la teneur en acide lactique par chromatographie liquide à haute performance. Les résultats sont représentés graphiquement sur la Figure 1 où les courbes 1 à 4 représentent la quantité Q d'acide lactique produit, exprimée en g/l du milieu de fermentation, en fonction de l'âge en heures (h) de la fermentation, selon les exemples 1 à 4 respectivement.

Exemples 5 et 6

On réalise deux fermentations dans les conditions décrites à l'exemple 1 à l'aide de la même culture, avec les différences suivantes :

|  | Exemple 5 | Exemple 6 |
|---|---|---|
| Amidon fluidifié par α amylase | 6,50 kg | 7,25 kg |
| Glucoamylase | 15,0 ml | 16,7 ml |
| CaCO$_3$ | 4,0 kg | 4,5 kg |

Les résultats sont répertoriés dans le Tableau 1.

Tableau 1

ACIDE LACTIQUE D-

| N° ESSAIS | SOURCE CARBONEE | | | GLUCOAMYLASE ml/l | SOURCE AZOTEE | | AGENT DE SALIFICATION | ACIDE LACTIQUE PRODUIT g/l | DUREE DE DE LA FERMENTA-TION (heures) | PRODUCTI-VITE g/l/h |
|---|---|---|---|---|---|---|---|---|---|---|
| | GLUCOSE g/l | HYDROLYSAT D'AMIDON (1) g/l | AMIDON g/l | | CSL (2) g/l | PROTEOLYSAT DE POISSON g/l | | | | |
| 1 | | | 135 | 0,312 | 35 | 5,4 | CaCO$_3$ | 123,3 | 45 | 2,74 |
| 2 | 130 | | | | 35 | 5,4 | CaCO$_3$ | 121 | 85 | 1,42 |
| 3 | | 183 | | | 35 | 5,4 | CaCO$_3$ | 121,3 | 107 | 1,13 |
| 4 | | | 135 | | 35 | 5,4 | CaCO$_3$ | 91,5 | 65 | 1,40 |
| 5 | | | 130 | 0,30 | 35 | 5,4 | CaCO$_3$ | 113 | 40 | 2,82 |
| 6 | | | 145 | 0,337 | 35 | 5,4 | CaCO$_3$ | 134,6 | 60 | 2,24 |

(1) Contenu en glucose 71 %
(2) CSL = "corn steep liquor" = eau de lavage de maïs concentrée

EP 0 354 828 A1

Exemple 7 - Production d'acide lactique L+

Dans un fermenteur contenant 7 kg d'amidon de blé fluidifié dans les conditions décrites à l'exemple 1, on ajoute 1,750 kg d'eau de lavage de maïs concentrée, 0,270 kg de protéolysat de poisson, 25 ml d'acide phosphorique. Le mélange est dilué à l'eau potable à un volume de 27 litres, agité, ajusté à pH 6,0 par NaOH et stérilisé par injection de vapeur pendant 1 heure.

On ajoute 16 litres d'une solution stérile aqueuse contenant 4,35 kg de carbonate de calcium. La température est ajustée à 40°C. Le milieu est additionné de 16,1 ml d'une préparation enzymatique contenant une glucoamylase (AMG 200L® - NOVO Industry) et ensemencé par 1,7 litres d'une culture de Lactobacillus casei IFO 3425 préalablement incubé dans un milieu MRS.

Le milieu de fermentation (volume final 50 litres) est agité et incubé sous atmosphère d'azote stérile à 40°C.

La teneur en acid lactique L+ est déterminée périodiquement par méthode HPLC.

| Age de la fermentation | 20 h | 40 h | 60 h | 80 h | 107 h |
|---|---|---|---|---|---|
| Acide lactique L+ | 21 g/l | 47,5 g/l | 66 g/l | 98 g/l | 120,2 g/l |

Exemples 8 à 11

Dans un fermenteur de capacité utile 50 litres, on introduit 6 kg d'amidon. La mise en suspension est effectuée à l'eau potable sous agitation dans un volume total de 27 litres.

Le pH de la suspension d'amidon est ajusté à 6,5 par $H_2SO_4$ et l'on ajoute au milieu, 3,25 ml d'une préparation enzymatique contenant une $\alpha$ amylase thermostable (TERMAMYL 120L® - NOVO Industry).

La suspension d'amidon est ensuite fluidifiée en chauffant par injection de vapeur pendant 30 mn à 100°C.

La solution d'amidon fluidifié est refroidie, additionnée de 1,750 kg de corn steep liquide, 0,270 kg de protéolysat de poisson, 25 ml d'acide phosphorique. Le volume est ajusté à 38 litres par de l'eau potable et le pH ajusté à 5,0 par NaOH.

La solution ainsi obtenue est stérilisée par injection de vapeur pendant 1 h à 100°C. Le volume au terme de cette étape est, après refroidissement à 40°C, égal à 50 litres.

Le milieu est additionné de 13,8 ml d'une préparation enzyma tique contenant une gluco amylase (AMG 200L® - NOVO Industry) et ensemencé par 1,7 litre d'une culture préliminaire de Lactobacillus lactis ATCC 12 314 dans un milieu MRS convenablement incubé.

La préparation est agitée et incubée à 40°C sous atmosphère d'azote stérile.

Le pH est maintenu à 6,0 par addition automatique d'un agent de salification : $NH_3$, $NH_4OH$, NaOH ou KOH selon les exemples 8 à 11 respectivement.

La teneur en acide lactique D- déterminée par méthode HPLC est la suivante :

| Age de la fermentation : Acide lactique D (g/l) : | 25 h | 50 h | 75 h | 85 h | 90 h |
|---|---|---|---|---|---|
| Exemple 8 (NH₃) | 39 | 76 | 98 | 101,8 | 102,3 |
| Exemple 9 (NH₄OH) | 35 | 71 | 91 | 93,5 | 94,5 |
| Exemple 10 (NaOH) | 30 | 68 | 87 | 89,9 | |
| Exemple 11 (KOH) | 31 | 71 | 88 | 93,3 | |

**Revendications**

1. Procédé de production d'acide lactique par fermentation au moyen de microorganismes d'un milieu nutrifif aqueux contenant de l'amidon comme source de carbone assimilable caractérisé en ce que la fermentation est conduite en présence additionnelle d'au moins une enzyme amylolytique saccharifiante.

2. Procédé selon la revendication 1 caractérisé en ce que l'amidon est mis en oeuvre sous forme liquéfiée ou partiellement saccharifiée.

3. Procédé selon la revendication 1 caractérisé en ce que l'amidon est mis en oeuvre sous forme brute.

4. Procédé selon la revendication 3 caractérisé en ce que le milieu de fermentation contient une

EP 0 354 828 A1

enzyme de liquéfaction en addition à l'enzyme saccharifiante.

5. Procédé selon l'une des revendications 1 à 4 caractérisé en ce que l'amidon est présent en quantité nécessaire pour fournir 0,9 à 18 % en poids de glucose par rapport au milieu de fermentation.

6. Procédé selon l'une des revendications 1 à 5 caractérisé en ce que l'enzyme saccharifiante est choisie parmi les α amylases, les β amylases, la glucoamylase, l'isoamylase, la pullulanase et leurs mélanges.

7. Procédé selon l'une des revendications 1 à 6 caractérisé en ce que l'enzyme saccharifiante est utilisée en quantité suffisante pour fournir 0,04 à 2 unités d'activité enzymatique, de préférence 0,1 à 1 unité, par g d'amidon exprimé en matières sèches.

8. Procédé selon l'une des revendications 1 à 7 caractérisé en ce que l'enzyme saccharifiante est une glucoamylase et est utilisée en quantité comprise entre 0,02 et 1 %, de préférence entre 0,05 et 0,5 %, en poids basé sur le poids de solides contenu dans l'amidon.

9. Procédé selon l'une des revendications 1 à 8 caractérisé en ce que le microorganisme est choisi parmi les microorganismes appartenant au genre Lactobacillus, au genre Streptococcus, au genre Pediococcus, au genre Bacillus et Sporolactobacillus et les champignons apparentant au genre Rhizopus.

10. Procédé selon l'une des revendications 1 à 9 caractérisé en ce que la fermentation est réalisée à un pH compris entre 3,0 et 8,0 de préférence entre 5,0 et 7,0.

11. Procédé selon la revendication 10 caractérisé en ce que la valeur du pH est contrôlée à l'aide d'un additif choisi parmi les hydroxydes ou carbonates alcalins, alcalino-terreux ou d'ammonium.

7

FIGURE 1

| | Office européen des brevets | **RAPPORT DE RECHERCHE EUROPEENNE** | Numero de la demande |
|---|---|---|---|

EP  89 40 2136

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | US-A-2 588 460  (R.S. ARIES) <br> * En entier * | 1-6 | C 12 P   7/56 |
| Y | | 9 | |
| Y | CHEMICAL ABSTRACTS, vol. 85, no. 23, 6 décembre 1976, page 385, résumé no. 175613j, Columbus, Ohio, US; & JP-A-76 88 691 (TOHO SAKE BREWERY CO., LTD) 03-08-1976 <br> * Résumé * | 9 | |
| Y | CHEMICAL ABSTRACTS, vol. 109, no. 5, 1er août 1988, page 501, résumé no. 36531g, Columbus, Ohio, US; T.R. SHAMALA et al.: "Fermentation of starch hydrolysates by Lactobacillus plantarum", & J. IND. MICROBIOL. 1988, 3(3), 175-8 <br> * Résumé * | 9 | |
| X | CHEMICAL ABSTRACTS, vol. 109, no. 9, 29 août 1988, page 565, résumé no. 71949w, Columbus, Ohio, US; B. MASLOWSKI: "Use of the enzymic preparation Amylopol P in the preparation of a starch substrate for lactic acid fermentation", & PR. NAUK. AKAD. EKON. IM. OSKARA LANGEGO WROCLAWIU 1987, 397, 107-11 <br> * Résumé * <br><br> ---                              -/- | 1-7 | **DOMAINES TECHNIQUES RECHERCHES (Int. Cl.5)** <br><br> C 12 P |

Le présent rapport a été établi pour toutes les revendications

| Lieu de la recherche | Date d'achèvement de la recherche | Examinateur |
|---|---|---|
| LA HAYE | 15-09-1989 | PULAZZINI A.F.R. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

........................................................................

& : membre de la même famille, document correspondant

EPO FORM 1503 03.82 (P0402)

Office européen
des brevets

**RAPPORT DE RECHERCHE EUROPEENNE**

Numero de la demande

EP 89 40 2136

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | BIOTECHNOLOGY AND BIOENGINEERING, vol. 31, no. 2, 5 février 1988, pages 183-187, John Wiley & Sons, Inc., New York; H. KUROSAWA et al.: "L-lactic acid production from starch by coimmobilized mixed culture system of aspergillus awamori and streptococcus lactis" * Résumé * | 1-7 | |
| Y | IDEM --- | 9 | |
| A | CHEMICAL ABSTRACTS, vol. 108, no. 13, 28 mars 1988, page 489, résumé no. 110802u, Columbus, Ohio, US; T.R. SHAMALA et al.: "Degradation of starchy substrates by a crude enzyme preparation and utilization of the hydrolyzates for lactic fermentation", & ENZYME MICROB. TECHNOL. 1987, 9(12), 726-9 ----- | | |

DOMAINES TECHNIQUES RECHERCHES (Int. Cl.5)

Le présent rapport a été établi pour toutes les revendications

| Lieu de la recherche | Date d'achèvement de la recherche | Examinateur |
|---|---|---|
| LA HAYE | 15-09-1989 | PULAZZINI A.F.R. |

EPO FORM 1503 03.82 (P0402)